## Europäisches Patentamt
## European Patent Office
## Office européen des brevets

(11) Veröffentlichungsnummer: **0 036 556**
**B1**

(12) **EUROPÄISCHE PATENTSCHRIFT**

(45) Veröffentlichungstag der Patentschrift:
08.08.84

(21) Anmeldenummer: 81101746.6

(22) Anmeldetag: 10.03.81

(51) Int. Cl.³: **C 22 C 5/04**, C 22 C 30/00,
**A 61 K 6/04**

(54) Goldfreie Legierungen zum Aufbrennen keramischer Massen.

(30) Priorität: 13.03.80 DE 3009650

(43) Veröffentlichungstag der Anmeldung:
30.09.81 Patentblatt 81/39

(45) Bekanntmachung des Hinweises auf die Patenterteilung:
08.08.84 Patentblatt 84/32

(84) Benannte Vertragsstaaten:
CH FR GB IT LI SE

(56) Entgegenhaltungen:
DE - B - 1 533 234
DE - C - 684 186
DE - C - 714 820

(73) Patentinhaber: Degussa Aktiengesellschaft,
Weissfrauenstrasse 9, D-6000 Frankfurt am Main 1 (DE)

(72) Erfinder: Zwingmann, Gerhard, Dr., Haagstrasse 77,
D-6454 Bruchköbel (DE)

BUNDESDRUCKEREI BERLIN

## Beschreibung

Die Erfindung betrifft goldfreie Legierungen zum Aufbrennen von keramischen Massen, insbesondere für dentaltechnische Zwecke.

Seit vielen Jahren werden goldhaltige Edelmetall-Legierungen mit hohen Goldgehalten als Material für mit Dentalporzellan überzogenem Zahnersatz verwendet. Diese bekannten Legierungen eignen sich sehr gut für diesen Zweck und führen zu gut haftenden, normalerweise rißfreien Porzellanüberzügen. Wegen ihrer relativ niedrigen Schmelztemperaturen sind sie gut verarbeitbar und wegen ihres hohen Edelmetall-Anteils sehr korrosionsbeständig.

Infolge der hohen Goldpreise und der hohen Dichte dieser bekannten Legierungen sind bisher viele Versuche unternommen worden, diese hochgoldhaltigen Legierungen durch solche auf der Basis Silber-Palladium oder auf Unedelmetallbasis zu ersetzen. Solche Unedelmetall-Legierungen enthielten bisher stets Nickel als Hauptbestandteil, daneben in vielen Fällen noch Beryllium als Legierungsbestandteil. Beide Elemente sind jedoch toxisch und daher für die Verwendung im menschlichen Mund wenig geeignet.

Daneben weisen diese Nickellegierungen im allgemeinen hohe Solidus-Temperaturen auf, was ihre Verwendung in Dental-Labors sehr erschwert. Außerdem besitzen sie gegenüber hochgoldhaltigen Legierungen eine verringerte Korrosionsbeständigkeit, die allerdings durch hohe Chromanteile verbessert werden kann. Chromzusätze führen aber beim Aufbrennen von Porzellan sehr leicht zu grünlichen Verfärbungen des Porzellans, die unerwünscht sind.

Die gleichen Nachteile zeigen auch die bekannten Aufbrennlegierungen auf der Basis von Silber-Palladium.

In neuerer Zeit sind Unedelmetall-Legierungen auf Kobalt-Basis zum Aufbrennen von Dentalporzellanen bekannt geworden, die keine bzw. nur unwesentliche Mengen an Edelmetallen enthalten. Da Kobalt aber im Gegensatz zu Nickel erst bei Chromgehalten von mehr als 25 Gew.-% korrosionsbeständig wird, macht sich auch bei diesen Legierungen die Grünfärbung des Porzellans beim Aufbrennen bemerkbar. Außerdem ist bei diesen Legierungen die Haftbeständigkeit des aufgebrannten Porzellans nicht ausreichend.

In der DE-C-684 186 wird eine Dentalgußlegierung beschrieben, die neben 20 bis 60% Palladium noch bis zu 15% Chrom, Rest Kobalt enthalten kann. Die Verwendung dieser Legierung zum Aufbrennen von keramischen Massen ist nicht erwähnt.

Es war daher Aufgabe der vorliegenden Erfindung, goldfreie Legierungen zum Aufbrennen von keramischen Massen, insbesondere für dentaltechnische Zwecke, zu finden, die keine toxischen Bestandteile enthalten, korrosionsbeständig sind, einen relativ niedrigen Schmelzpunkt besitzen, beim Aufbrennen keine Verfärbung der keramischen Massen bewirken und eine haftfeste Verbindung gewährleisten.

Diese Aufgabe wurde erfindungsgemäß dadurch gelöst, daß man Legierungen verwendet, die 25—35 Gew.-% Palladium, 5—15 Gew.-% Chrom, 5—15 Gew.-% Molybdän und/oder Wolfram, 2—6 Gew.-% Cer, 0,2—0,8 Gew.-% Bor, Rest Kobalt enthalten.

Durch den Borzusatz wird die Schmelztemperatur der ternären Palladium-Kobalt-Nickel-Legierungen erniedrigt und dadurch ihr Gießverhalten verbessert. Die Schmelztemperatur der Legierungen mit hohem Palladiumgehalt wird dabei durch den Borzusatz stärker erniedrigt, so daß mit steigendem Palladiumgehalt in den Legierungen der Borzusatz vermindert werden kann.

Mit dem Zusatz von 5 bis 15 Gew.-% Molybdän und/oder Wolfram erreicht man eine bessere Einstellung der Härte, der Zugfestigkeit, der Bruchdehnung und in gewissem Maße des thermischen Ausdehnungskoeffizienten.

Eine gute Haftfestigkeit der keramischen Massen auf den metallischen Aufbrennlegierungen kann nur erreicht werden, wenn die thermischen Ausdehnungskoeffizienten von keramischer Masse und Legierung in etwa übereinstimmen. Außerdem ist die Ausbildung einer gewissen Haftoxidschicht auf der Legierung beim Aufbrennen der keramischen Masse notwendig. Legierungen mit hohen Kobaltgehalten und relativ geringen Chrom-Anteilen tendieren bei höheren Temperaturen, wie sie zum Aufbrennen von Dentalporzellan erforderlich sind, zur Ausbildung relativ dicker Zunderschichten, die die Haftfestigkeit der Porzellanschichten fallweise beeinträchtigen können.

Durch Zusatz von 2 bis 6 Gew.-% Cer bzw. der sonstigen Seltenen Erden, wie Scandium, Yttrium oder Lanthan, wird die Ausbildung der Zunderschicht stark gehemmt. Durch diese Zusätze wird außerdem die zum Teil vorhandene Neigung zur Rißbildung in den aufgebrannten keramischen Massen herabgesetzt.

Die erfindungsgemäßen Legierungen lassen sich mit allen handelsüblichen keramischen Massen für dentaltechnische Zwecke verblenden, mit guter Haftfestigkeit und ohne daß Rißbildung oder eine Verfärbung auftritt. So wurden beispielsweise aus einer erfindungsgemäßen Legierung mit 45,6% Co, 30% Pd, 10% Cr, 10% Mo, 4% Ce und 0,4% B, aus einer handelsüblichen hochgoldhaltigen Aufbrennlegierung, sowie aus einer handelsüblichen Unedelmetall-Aufbrennlegierung auf Nickelbasis jeweils 50 Zahnkronen hergestellt und mit einer handelsüblichen Porzellanmasse bei 950°C überzogen. Keine der Kronen aus der erfindungsgemäßen Legierung zeigte nach dem Aufbrennen Sprünge im Porzellan, während bei der hochgoldhaltigen Legierung zwei und bei der Nickellegierung fünf Sprünge auftra-

2

ten. Auch nach längerer Lagerzeit wiesen die Kronen aus der erfindungsgemäßen Legierung keine sogenannten Spätsprünge auf.

Die Tabelle zeigt die Eigenschaften einiger erfindungsgemäßen Legierungen im Vergleich zu bekannten hochgoldhaltigen und nickelhaltigen Aufbrennlegierungen. Die Korrosionsmessungen wurden dabei potentiostatisch in 0,1 n Kochsalzlösungen durchgeführt und das Durchbruchspotential $E_L$ bestimmt.

Zugfestigkeit, Bruchdehnung und Härte wurden an im Schleuderguß mit induktiver Erhitzung hergestellten Stäben gemessen. Zur Bestimmung der Haftfestigkeit des Porzellans auf dem Unterlage-Material wurden Blechstreifen aus der Legierung einseitig mit Porzellan überzogen, darauf ein zweiter Blechstreifen mit einem hochfesten Kleber aufgebracht und dieser Verbund in einer Zugprüfmaschine getestet.

| | Legierung | Durch-bruchs-poten-tial $E_L$ [nV] | Zug-festig-keit $\sigma_B$ [N/mm²] | Bruch-deh-nung $\delta$ % | Härte HV 3 | Therm. Ausdehn. Koeff. $\alpha$ (10—500°C) · $10^{-6}$ | Haft-festig-keit [N/mm²] | Auf-brenn-ver-halten |
|---|---|---|---|---|---|---|---|---|
| 1 | 60 Co 10 Cr 30 Pd (Stand der Technik) | +350 | 710 | 10 | 290 | 14,0 | | befried. |
| 2 | 45,6 Co 10 Cr 30 Pd 10 Mo 4 Ce 0,4 B | +350 | 880 | 7 | 350 | 15,1 | 5,1 | sehr gut |
| 3 | handelsübliche hoch-goldhaltige Legierung | +450 | 650 | 7 | 240 | 14,5 | 3,1 | gut |
| 4 | handelsübliche nickel-haltige Legierung | + 35 | 700 | 5 | 320 | 14,5 | 3,8 | befried. |

**Patentanspruch**

Goldfreie Legierung zum Aufbrennen von keramischen Massen insbesondere für dentaltechnische Zwecke, dadurch gekennzeichnet, daß sie aus 25—35 Gew.-% Palladium, 5—15 Gew.-% Chrom, 5—15 Gew.-% Molybdän und/oder Wolfram, 2—6 Gew.-% Cer, 0,2—0,8 Gew.-% Bor, Rest Kobalt besteht.

**Claim**

A gold-free alloy for bonding of ceramic material by firing, in particular for dental purposes, characterised in that the alloy consists of from 25—35%, by weight, of palladium, from 5—15%, by weight, of chromium, from 5—15%, by weight, of molybdenum and/or tungsten, from 2—6%, by weight, of cerium, from 0.2 to 0.8%, by weight, of boron and the remainder cobalt.

**Revendication**

Alliage exempt d'or pour l'application par cuisson de masses céramiques, en particulier pour des applications dentaires caractérisé en ce qu'il est constitué de 25 à 35% en poids de palladium, 5 à 15% en poids de chrome, 5 à 15% en poids de molybdène et/ou de tungstène, 2 à 6% en poids de cérium, 0,2 à 0,8% en poids de bore, le reste étant du cobalt.